# EUROPÄISCHE PATENTSCHRIFT

(11) **EP 0 854 853 B1**
(45) Veröffentlichungstag und Bekanntmachung des Hinweises auf die Patenterteilung: **31.10.2001**
(21) Anmeldenummer: 97925040.4
(22) Anmeldetag: 04.06.1997
(51) Int. Cl.: C07C 68/06, C07C 69/96

(54) **VERFAHREN ZUR HERSTELLUNG VON DIALKYLCARBONATEN**
PROCESS FOR PRODUCING DIALKYL CARBONATES
PROCEDE DE PREPARATION DE CARBONATES DE DIALKYLE

(30) Priorität: 13.06.1996 DE 19623508
(43) Veröffentlichungstag der Anmeldung: 29.07.1998
(73) Patentinhaber: Cognis Deutschland GmbH, 40589 Düsseldorf (DE)
(72) Erfinder: REUTER, Erich, D-40591 Düsseldorf (DE); KNÖRR, Walter, D-89257 Illertissen (DE); GUTSCHE, Bernhard, D-40724 Hilden (DE)
(86) Internationale Anmeldenummer: EP9702891
(87) Internationale Veröffentlichungsnummer: WO9747583

(56) Entgegenhaltungen:
- EP-A- 0 530 615
- EP-A- 0 569 812
- WO-A-92/10462

## Beschreibung

Die Erfindung befindet sich auf dem Gebiet der kosmetischen Rohstoffe und betrifft ein neues Verfahren zur Herstellung von Dialkylcarbonaten.

### Stand der Technik

Niedere Alkylcarbonate kann man durch die Umsetzung von Ethylen und Propylenglykolcarbonaten (Glykolcarbonaten) mit Alkoholen in Gegenwart von Katalysatoren erhalten. Obwohl diese Reaktion mit hoher Selektivität ablaufen kann, weisen die Verfahren bei ihrer Durchführung eine Reihe von Nachteilen auf. In der Regel verläuft die Umesterung bei Normaldruck relativ langsam, so dass die Anwendung erhöhter Temperatur, vielfach oberhalb des Siedepunktes des eingesetzten Alkohols, empfohlen wird, woraus eine Durchführung in Druckgefäßen resultiert **(EP 0001082 A1, EP 00001083 A1)**. Hierbei verläuft die Umsetzung normalerweise nur bis zur Gleichgewichtseinstellung der Umesterungsreaktion. Nach der Entnahme aus dem Druckbehälter muß das Reaktionsgemisch sehr rasch vom Katalysator entfernt werden, beispielsweise durch eine Flashdestillation, damit sich nicht in Umkehrung der Bildungsreaktion, etwa beim Abdestillieren des leichter siedenden Alkohols, die Ausgangsverbindungen zurückbilden. Beim Abdestillieren vom Katalysator kann sich das noch im Gleichgewicht befindliche Glykolcarbonat zu Kohlendioxid und zu Polyglykolen zersetzen, geht damit für eine weitere Umesterung unter Ausbeuteschmälerung verloren, und alle genannten Nebenprodukte stören die Aufarbeitung. Aber auch wenn diese Abtrennung des Katalysators zufriedenstellend gelingt, müssen noch mehrere Destillationen durchgeführt werden. So ist zuerst eine Trennung von Hochsiedern von den Niedrigsiedern erforderlich. Die destillative Reinigung von Ethylenglykol von unvollständig umgesetztem Ethylenglykolcarbonat, welches bevorzugt wiederum für die Herstellung der Dialkylcarbonate eingesetzt wird, ist jedoch nicht ohne Einschränkung möglich, da beide Verbindungen ein Azeotrop bilden. Eine ähnliche Schwierigkeit findet man vor, wenn man Methanol, welches die bevorzugte alkoholische Komponente ist, von dem gebildeten Dimethylcarbonat abzutrennen hat. Auch diese Verbindungen bilden ein Azeotrop, das nur umständlich auseinander zu destillieren ist **(EP 0000894 A1)**. Die Herstellung höherer Carbonate erfolgt häufig auch durch Umsetzung kurzkettiger Carbonate wie Diethylcarbonat, Dimethylcarbonat, Dibutylcarbonat und/oder Dipropylcarbonat diskontinuierlich mit Alkoholen in einem Rührkessel. Während der Reaktion wird der entstehende Alkohol (Methanol, Ethanol, Propanol, Butanol) abdestilliert und damit die Gleichgewichtslage zur Produktseite hin verschoben. Der Produktalkohol kann nicht in reiner Form abdestilliert werden, es fällt immer ein Gemisch aus Alkohol und kurzkettigem Eduktcarbonat an. Beim Einsatz von Dimethylcarbonat erhält man im günstigsten Fall ein Gemisch mit azeotroper Zusammensetzung (ca. 30 Gew.-% Dimethylcarbonat und 70 Gew.-% Methanol). Dieses Gemisch kann nur mit dem sehr aufwendigen Verfahren der Azeotroprektifikation getrennt werden.

Die Umesterung von kurzkettigen Dialkylcarbonaten mit langkettigen Fettalkoholen ist grundsätzlich aus der **WO 92/10462** bekannt. Aus der **DE 4129316 A1** ist ein Verfahren zur Umesterung von Glycolcarbonaten mit niederen Alkoholen bekannt, bei dem man niedere Alkylcarbonate erhält. Bei diesem Verfahren wird die Bildung eines Azeotrops vermieden und die Umsetzung erfolgt unter milden Bedingungen. Schließlich sei auch auf die **EP 0530615 A2** verwiesen, in der die Umesterung von Ethylen- bzw. Propylencarbonat mit Methanol oder Ethanol beschrieben wird.

Die Aufgabe der vorliegenden Erfindung bestand darin, ein einfaches Verfahren zur Herstellung höherer Dialkylcarbonate, zu entwickeln. Eine weitere Aufgabe war es, die Herstellung höherer Dilakylcarbonate mit einem hohen Anteil symmetrischer Dialkylcarbonate zu ermöglichen.

### Beschreibung der Erfindung

Gegenstand der Erfindung ist ein Verfahren zur Herstellung von Dialkylcarbonaten der Formel **(I)**

**R**^{**1**}**O-CO-OR**^{**2**} **(I)**

in der R¹ und R² für einen linearen oder verzweigten, gesättigten, ungesättigten und/oder alkoxylierten Alkylrest stehen und R¹ und R² gleich oder verschieden sein können, durch Umesterung von Di-C₁-C₄-alkylcarbonaten mit aliphatischen, linearen oder verzweigten Alkoholen mit 6 bis 22 Kohlenstoffatomen in Gegenwart eines Katalysators, welches sich dadurch auszeichnet, dass man die Umesterung in einer mit Füllkörpern oder Einbauten ausgerüsteten Reaktionskolonne durchführt und die Reaktionspartner im Gegenstrom führt.

Überraschenderweise wurde festgestellt, dass durch Umesterung von niederen Dialkylcarbonaten mit höheren Alkoholen, wobei die Edukte im Gegenstrom geführt wurden, höhere Dialkylcarbonate, insbesondere symmetrische Dialkylcarbonate, in hohen Ausbeuten erhalten werden konnten und gleichzeitig das Problem der Aufarbeitung eines Azeotrops vermieden werden konnte.

### Dialkylcarbonate

Als Edukte lassen sich Dialkylcarbonate der Formel **(I)** einsetzten, in der R¹ und R² vorzugsweise gleich sind und für einen Kohlenwasserstoffrest mit 1 bis 4 C-Atomen, insbesondere für einen linearen, gesättigten Kohlenwasserstoffrest stehen, besonders bevorzugt handelt es sich um Diethyl- bzw. Dimethylcarbonat.

### Alkohole

Die Umesterung der Dialkylcarbonate der Formel **(I)** erfolgt mit Alkoholen der Formel **(II)**,

**R**^{**3**}**OH (II)**

wobei es sich bevorzugt um Guerbetalkohole, besonders bevorzugt um Fettalkohole handelt. Unter Fettalkoholen sind primäre aliphatische Alkohole der Formel **(II)** zu verstehen, in der R³ für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, 2-Butyl-1-Octanol, Laurylalkohol, Isotridecylalkohol, Myristylalkohol, Cetylalkohol, 2-Hexyl-1-decanol, Palmoleylalkohol, Stearylalkohol, Isostearylalkohol, Oleylalkohol, Elaidylalkohol, Petroselinylalkohol, Linolylalkohol, Linolenylalkohol, 2-Octyl-1-dodecanol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Unter Guerbetalkoholen sind Alkohole der Formel **(II)** zu verstehen, wobei der Rest R³ für einen verzweigten Alkylrest mit 12 bis 44, bevorzugt 12 bis 22 Kohlenstoffatomen steht. In einer weiteren Ausführungsform können ethoxylierte Alkohole eingesetzt werden, wobei es sich bevorzugt um die oben aufgeführten Alkohole mit 1 bis 20, vorzugsweise 2 bis 10 Ethylen- und/oder Propylenoxideinheiten pro Molekül handelt. Besonders bevorzugt werden gemäß der vorliegenden Erfindung 2-Ethylhexylalkohol, 2-Butyl-1-octanol, 2-Hexyl-1-decanol, 2-Octyl-1-dodecanol als Alkoholkomponenten eingesetzt.

### Katalysatoren

Erfindungsgemäß lassen sich alle üblichen Umesterungskatalysatoren verwenden, wie sie zum Beispiel in der **DE 4129316 A1** aufgeführt sind. Besonders bevorzugt werden Natriummethanolat und Tetraisopropyltitanat, Tetra-n-butyltitanat und Tetrat-2-ethylhexyltitanat eingesetzt. Üblicherweise setzt man den Katalysator in Mengen von 0,01 bis 2,5 insbesondere von 0,05 bis 0,5 Gew.-% bezogen auf die Eduktmasse ein.

Erfindungsgemäß kann man die Umesterung der Dialkylcarbonate sowohl kontinuierlich, als auch in einem Semi-Batch Verfahren durchführen. Bei Produktionsmengen kleiner als 1000 t/a wird bevorzugt im Semi-Batch Verfahren gearbeitet, während größere Mengen vorzugsweise kontinuierlich hergestellt werden.

### Kontinuierliches Verfahren

Die kontinuierliche Umesterung von Dialkylcarbonaten erfolgt üblicherweise in einer mit Füllkörpern und/ oder Einbauten ausgerüsteten Reaktionskolonne wie sie auch in der **EP 0033929 A1** und der **DE 3146142 A1** beschrieben sind. Die zu verwendenden Füllkörper bzw. geordneten Packungen sind die für Destillationen an sich üblichen, wie sie beispielsweise in **Ullmann's Encyclopädie der Techn. Chemie, 4. Auflage, Band 2, Seite 528 ff** oder in den Firmenschriften der in Frage kommenden Apparatebaufirmen beschrieben sind. Beispielsweise seien genannt: Raschig- oder Pallringe, Berl-Intalex- oder Torussättel, Interpackkörper aus verschiedenen Materialien, wie Glas, Steinzeug, Porzellan, Kohlenstoff, Edelstahl, Kunststoff, die insbesondere bei der Verwendung von Metallgewebe oder maschenartig verarbeitet sein können. Bevorzugt sind Füllkörper und geordnete Packungen, die eine große Oberfläche und eine gute Benetzung sowie eine ausreichende Verweilzeit der Flüssigkeit aufweisen. Dies sind beispielsweise Pall- und Novolax-Ringe, Berlsättel, BX-Packungen, Montz-Pak, Mellapak, Melladur, Kerapak und CY-Packungen. Für das erfindungsgemäße Verfahren sind jedoch nicht nur Füllkörperkolonnen geeignet, sondern auch solche mit festen Einbauten. Hierunter sind solche mit Glocken- bzw. Ventilböden mit hohen Verweilzeiten bei gutem Stoffaustausch bevorzugt. Geeignet sind jedoch allgemein auch weitere Bodenkolonnen z. B. solche mit Sieb-, Glocken-, Ventil-, Tunnel- und Zentrifugalböden, die wiederum in unterschiedlichen Ausführungen vorliegen können. Typische Vertreter geeigneter Reaktionskolonnen sind weiterhin in der **EP 0033929 A1** sowie in der **DE 3146142 A1** beschrieben.

Besonders bevorzugt sind Reaktionskolonnen, bestehend aus zwei Segmenten, einem Reaktionsteil, mit speziellen Einbauten, insbesondere Glockenböden und einem reinen Stoffaustauschteil, mit einer geordneten Stoffaustauschpackung.

Die Kolonne wird bei Temperaturen von 60 bis 250 °C so betrieben, dass man vorzugsweise direkt unterhalb der Stoffaustauschpackung eine Lösung des Katalysators in dem zur Umesterung vorgesehenen Alkohol einspeist. Diese Lösung wird zuvor auf die entsprechende Reaktionstemperatur von 100 bis 200, vorzugsweise 120 bis 180 °C erwärmt. Das Eduktcarbonat wird im unteren Teil der Kolonne bei einer Temperatur von 150 bis 250, vorzugsweise 170 bis 210 °C, flüssig oder gasförmig eingespeist. Die eingesetzten molaren Mengen betragen von 1:2 bis 1:4 Mol Dialkylcarbonat zu dem entsprechenden Alkohol. Die Umesterung erfolgt in der flüssigen Phase auf den Kolonnenböden und das anfallende flüssige Produktcarbonat wird in der Kolonne nach unten abgeführt, während der entstehende Produktalkohol gasförmig am Kolonnenkopf abgezogen wird. Am Kolonnenkopf läßt sich reiner Produktalkohol abziehen während im Sumpf der Kolonne das Produktcarbonat gemeinsam mit dem überschüssigen Eduktalkohol und dem Katalysator anfällt. Der Sumpf läßt sich gemäß den aus dem Stand der Technik bekannten Verfahren aufarbeiten, um so das Produktcarbonat in der gewünschten Reinheit zu erhalten. Die Umsetzung des Eduktcarbonats liegt oberhalb von 99%, daher sind sowohl Destillat als auch Sumpf frei von Eduktcarbonat.

In einer weiteren Ausführungsform ist der Katalysator fest in der Reaktionskolonne angeordnet, das Verfahren wird wie oben bereits dargestellt durchgeführt, es entfällt lediglich die Zudosierung des im Alkohol gelösten Katalysators.

### Semibatch-Verfahren

Bei der Umesterung von Dialkylcarbonaten im Semibatch-Verfahren führt man die Reaktion vorzugsweise in einem Rührkessel mit aufgesetzter Kolonne durch. Bei der Kolonne kann es sich um die gleichen Typen wie für das kontinuierliche Verfahren beschrieben handeln. Bevorzugt ist auch hierbei eine Kolonne bestehend aus einem Reaktionsteil, insbesondere mit Glockenböden und einem Stoffaustauschteil. In dem Rührkessel wird das Eduktcarbonat mit einem Teil des Eduktalkohols und des Katalysators vorgelegt und anschließend unter Rühren auf eine Temperatur im Bereich von 150 bis 250, vorzugsweise 170 bis 210 °C, aufgeheizt. Ein Gemisch aus Eduktcarbonat und Produktalkohol verdampft in die Reaktionskolonne, in die gleichzeitig oberhalb der Reaktionszone Eduktalkohol und Katalysator (vorgewärmt auf die Reaktionstemperatur 80 bis 210, insbesondere 120 bis 180 °C) eingespeist werden. Die Reaktion verläuft analog dem kontinuierlichen Verfahren. Die molaren Mengen betragen in diesem Fall ebenfalls 1:2 bis 1:4 Mol Dialkylcarbonat pro Mol Alkohol. Im Rührkessel können 0 bis 100, vorzugsweise 50 bis 100 Gew.-% der umzusetzenden Menge des Dialkylcarbonats und 0 bis 90, vorzugsweise 50 bis 80 Gew.-%, der Menge des eingesetzten Alkohols vorgelegt werden, während die restlichen Anteile analog dem kontinuierlichen Verfahren in die Reaktionskolonne eingespeist werden.

Eine weitere Verfahrensvariante besteht darin, zunächst nur Eduktalkohol und Katalysator im Rührkessel vorzulegen und parallel zur Dosierung des übrigen Eduktalkohols in die Kolonne das Eduktcarbonat in den Rührkessel einzuspeisen.

### Beispiele

**Beispiele 1 bis 7.** Die Umsetzung von Dimethylcarbonat (DMC) mit 2-Ethylhexanol (2EH) erfolgte kontinuierlich in einer Glasreaktionskolonne mit einem Durchmesser von 80. Die Reaktionskolonne bestand aus 12 Glockenböden im Reaktionsteil und einer strukturierten Stoffaustauschpackung (Sulzer BX) in der Stoffaustauschzone. Der Reaktionsteil war mit einer Mantelheizung ausgestattet und wurde auf 120 bis 150 °C beheizt. Die Zugabe des Dimethylcarbonats erfolgte auf dem 6. Reaktionsboden, das 2-Ethylhexanol und das Natriummethanolat (Bsp.1 bis 6) bzw. Tetraisopropylorthotitanat (Bsp.7) wurden direkt unterhalb der Stoffaustauschzone zudosiert. Als Sumpfverdampfer diente ein Blasenverdampfer ohne Sumpfumwälzung. Die Ergebnisse sind in Tabelle 1 dargestellt. Angegeben sind die Anteile an symmetrischen (SYM) und unsymmetrischen Dialkylethern (US). Der Umsatz berechnet sich nach U =[SYM] / ([SYM] + [US]*0-5+DMC) * 100

**Beispiel 8.** Die Reaktion wurde in einem 41 Glasrührreaktor mit Doppelmantelheizung und aufgesetzter Kolonne durchgeführt. Die Kolonne setzte sich aus einem Reaktionsteil mit 5 Glockenböden und einem reinen Stoffaustauschteil mit strukturierter Packung (Sulzer EX, Höhe 300 mm) zusammen. Im Reaktor wurden 1800 g 2-Ethylhexanol und 10,8 g Natriummethanolat (30 %ig in Methanol) vorgelegt und erhitzt. Oberhalb des Reaktionsteils der Kolonne wurde kontinuierlich 144 ml pro Stunde 2-Ethylhexanol mit Katalysator zudosiert. Insgesamt wurden 650 ml 2-Ethylhexanol zugefügt. Gleichzeitig wurde Dimethylcarbonat mit 140 ml pro Stunde in den Reaktor eindosiert und zwar eine Gesamtmenge von 560 ml. Die Ergebnisse sind in Tabelle 2 dargestellt.

**Beispiele 9 bis 14.** Die Reaktion wurde in einem 4l Glasrührreaktor mit Doppelmantelheizung und aufgesetzter Kolonne durchgeführt. Die Kolonne setzte sich aus einem Reaktionsteil mit 10 Glockenböden (gesamtes Bodenvolumen 170 ml) und einem reinen Stoffaustauschteil mit strukturierter Packung (Sulzer DX, Höhe 270 mm) zusammen. Der Kolonnendurchmesser betrug 55 mm. Der Reaktionteil der Kolonne war als Doppelmantel ausgebildet und wurde mit Thermalöl beheizt. Im Reaktor wurden 2-Ethylhexanol und Katalysator vorgelegt und auf Reaktionstemperatur aufgeheizt. Oberhalb des Reaktionsteils der Kolonne wurde kontinuierlich 2-Ethylhexanol mit Katalysator zudosiert. Am Kopf der Kolonne wurde ein konstantes Rücklaufverhältnis von 1,0 eingestellt und das bei der Reaktion entstehende Methanol als Destillat abgezogen. Die Ergebnisse der Versuche sind ebenfalls in Tabelle 2 dargestellt.

**Beispiel 15 bis 16.** Die Versuche wurden in der Anlage von Beispiel 9 bis 14 durchgeführt. Als Alkohol wurde 2-Butyl-1-Octanol (DBO) eingesetzt. Die Fahrweise entsprach der von Beispiel 8 bis 14. Die Ergebnisse sind in Tabelle 3 dargestellt.

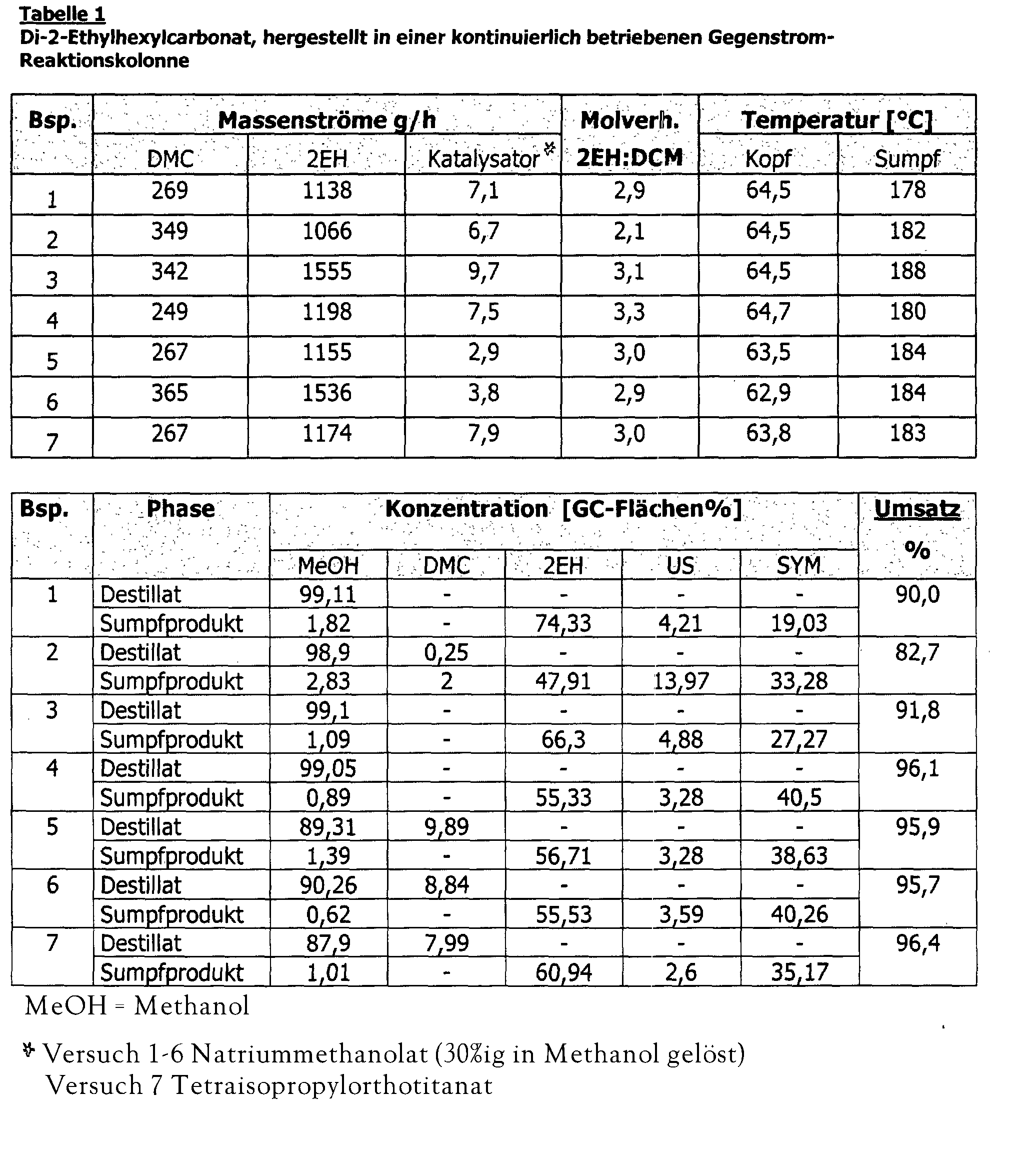

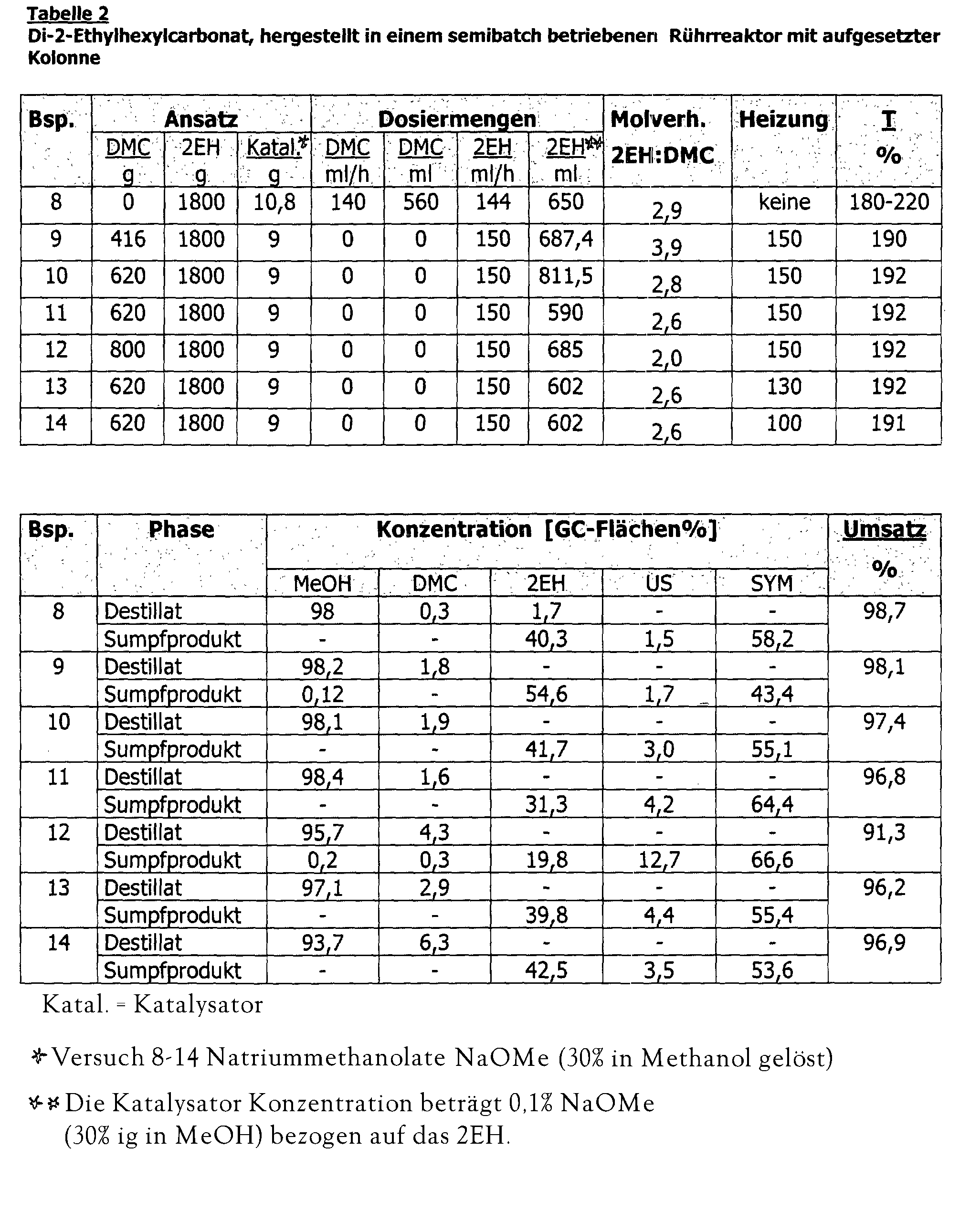

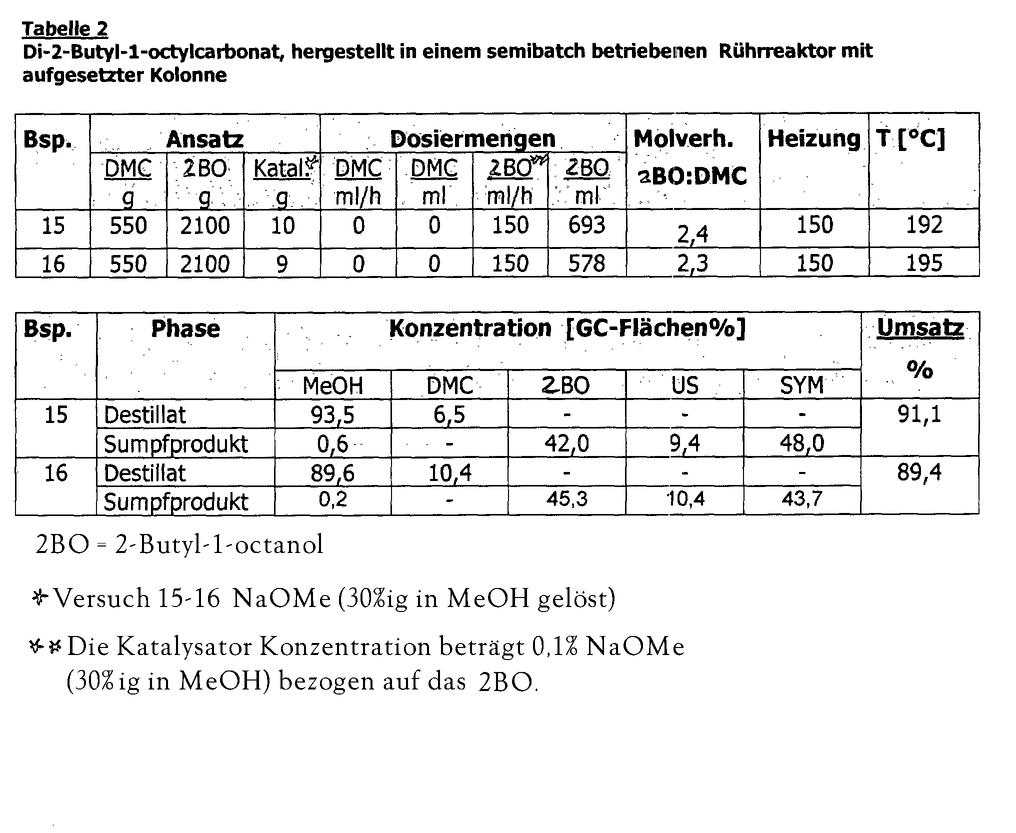

## Patentansprüche

1. Verfahren zur Herstellung von Dialkylcarbonaten der Formel (I)
**R**^{**1**}**O-CO-OR**^{**2**} **(I)**
in der R¹ und R² für einen linearen oder verzweigten, gesättigten, ungesättigten und/oder alkoxylierten Alkylrest stehen und R¹ und R² gleich oder verschieden sein können, durch Umesterung von Di-C₁-C₄-alkylcarbonaten mit aliphatischen, linearen oder verzweigten Alkoholen mit 6 bis 22 Kohlenstoffatomen in Gegenwart eines Katalysators, **dadurch gekennzeichnet, dass** man die Umesterung in einer mit Füllkörpern oder Einbauten ausgerüsteten Reaktionskolonne durchführt und die Reaktionspartner im Gegenstrom führt.

2. Verfahren nach Anspruch 1, **dadurch gekennzeichnet, dass** man die Umesterung in einer Reaktionskolonne bestehend aus einem unteren Reaktionsteil mit Glockenböden und einem darüber befindlichen mit Füllkörpern ausgestatteten Stoffaustauschteil durchführt.

3. Verfahren nach den Ansprüchen 1 und 2, **dadurch gekennzeichnet, dass** man als Edukte Dimethylcarbonat oder Diethylcarbonat einsetzt.

4. Verfahren nach den Ansprüchen 1 bis 3, **dadurch gekennzeichnet, dass** man als Alkohol Caprylalkohol oder 2-Ethylhexanol einsetzt.

5. Verfahren nach den Ansprüchen 1 bis 4, **dadurch gekennzeichnet, dass** man als Katalysator Natriummethanolat oder Tetraisopropylorthotitanat einsetzt.

6. Verfahren nach den Ansprüchen 1 bis 5, **dadurch gekennzeichnet, dass** man die Umesterung bei Temperaturen im Bereich von 60 bis 250 °C durchführt.

7. Verfahren nach den Ansprüchen 1 bis 6, **dadurch gekennzeichnet, dass** man pro Mol Dialkylcarbonat 1 bis 4 Mol Alkohol einsetzt.

8. Verfahren nach den Ansprüchen 1 bis 7, **dadurch gekennzeichnet, dass** man das Verfahren kontinuierlich durchführt und dabei den in dem Alkohol gelösten Katalysator direkt unterhalb der Stoffaustauschpackung zudosiert, während das Dialkylcarbonat im unteren Bereich der Kolonne flüssig oder gasförmig eingespeist wird.

9. Verfahren nach den Ansprüchen 1 bis 7,**dadurch gekennzeichnet, dass** man die Umesterung in einem Semi-Batch-Verfahren durchführt, wobei sich die Reaktionskolonne auf einem Rührkessel befindet, in dem 0 bis 100 Gew.-% des einzusetzenden Dialkylcarbonats in dem Rührkessel gemeinsam mit 0 bis 90 Gew.-% des umzusetzenden Alkohols vorgelegt werden, und die übrige Menge analog dem kontinuierlichen Verfahren in die Reaktionskolonne eingespeist werden.

## Claims

1. A process for the production of dialkyl carbonates corresponding to formula **(I):**
**R**^{**1**}**O-CO-OR**^{**2**} **(I)**
in which R¹ and R² may be the same or different and represent a linear or branched, saturated, unsaturated and/or alkoxylated alkyl group, by transesterification of di-C₁₋₄-alkyl carbonates with aliphatic, linear or branched alcohols containing 6 to 22 carbon atoms in the presence of a catalyst, **characterized in that** the transesterification reaction is carried out in a reaction column equipped with packings or internals through which the reactants flow in countercurrent to one another.

2. A process as claimed in claim 1, **characterized in that** the transesterification is carried out in a reaction column consisting of a lower reaction section with bubble trays and a vertically adjacent material transfer section equipped with packings.

3. A process as claimed in claims 1 and 2, **characterized in that** dimethyl carbonate or diethyl carbonate is used as educt.

4. A process as claimed in claims 1 to 3, **characterized in that** caprylic alcohol or 2-ethyl hexanol is used as the alcohol.

5. A process as claimed in claims 1 to 4, **characterized in that** sodium methanolate or tetraisopropyl orthotitanate is used as the catalyst.

6. A process as claimed in claims 1 to 5, **characterized in that** the transesterification is carried out at temperatures of 60 to 250°C.

7. A process as claimed in claims 1 to 6, **characterized in that** 1 to 4 moles of alcohol are used per mole of dialkyl carbonate.

## Revendications

1. Procédé pour la préparation de carbonates de dialkyle de formule (I)
R¹O-CO-OR² (I)
dans laquelle R¹ et R² représentent un radical alkyle linéaire ou ramifié, saturé, insaturé et/ou alcoxylé, et R¹ et R² peuvent être identiques ou différents, par transestérification de carbonates de dialkyle(C₁-C₄) avec des alcools aliphatiques, linéaires ou ramifiés, ayant de 6 à 22 atomes de carbone, en présence d'un catalyseur,
**caractérisé en ce qu'**
on effectue la transestérification dans une colonne de réaction garnie de corps de remplissage ou d'inserts et on envoie les partenaires réactionnels à contre-courant.

2. Procédé selon 1a revendication 1,
**caractérisé en ce qu'**
on effectue la transestérification dans une colonne de réaction constituée d'une partie inférieure de réaction munie de plateaux à cloches et d'une partie échangeuse de substances munie de corps de remplissage, se trouvant au-dessus de celle-ci.

3. Procédé selon les revendications 1 et 2,
**caractérisé en ce qu'**
on utilise comme produits de départ du carbonate de diméthyle ou du carbonate de diéthyle.

4. Procédé selon les revendications 1 à 3,
**caractérisé en ce qu'**
on utilise comme alcool l'alcool caprylique ou le 2-éthylhexanol.

5. Procédé selon les revendications 1 à 4,
**caractérisé en ce qu'**
on utilise comme catalyseur le méthanolate de sodium ou l'orthotitanate de tétra-isopropyle.

6. Procédé selon les revendications 1 à 5,
**caractérisé en ce qu'**
on effectue la transestérification à des températures dans la plage de 60 à 250°C.

7. Procédé selon les revendications 1 à 6,
**caractérisé en ce qu'**
on utilise 1 à 4 moles d'alcool par mole de carbonate de dialkyle.
